# EUROPEAN PATENT APPLICATION

(11) **EP 3 988 023 A1**
(43) Date of publication of application: **27.04.2022**
(21) Application number: 21204247.7
(22) Date of filing: 22.10.2021
(51) Int. Cl.: A61B 5/263, A61B 5/287, A61B 5/00, A61B 5/06, A61B 5/268

(54) **COATED ELECTRODES FOR INTRABODY LOCATION SENSING**

(30) Priority: 23.10.2020 US 202017079338
(71) Applicant: Biosense Webster (Israel) Ltd, Yokneam, 2066717 (IL)
(72) Inventor: GOVARI, Assaf, 2066717 Yokneam (IL); BEECKLER, Christopher Thomas, Irvine, 92618 (US)
(74) Representative: Carpmaels & Ransford LLP

(57) **Abstract**

A method includes obtaining a measurement of an electrogram voltage between a first electrode and a second electrode disposed within a body of a subject. The method further includes, while obtaining the measurement, causing (i) a first current, which has a first frequency, to flow between the first electrode and one or more reference electrodes, and (ii) a second current, which has a second frequency, to flow between the second electrode and the reference electrodes, such that a beat voltage, which has a third frequency that is a difference between the first frequency and the second frequency and is within a frequency band of the electrogram voltage, is seen at the first electrode and the second electrode. The first electrode and second electrode are coated with an output-impedance-reducing coating that facilitates obtaining the measurement despite the beat voltage.

## Description

### FIELD OF THE INVENTION

The present invention relates to intrabody probes for electrophysiological sensing.

### BACKGROUND

US Patent 8,615,287 discloses methods and systems for determining information about a position of an object within a distribution of materials having different complex conductivities. The method includes: (i) causing current to flow in the distribution; (ii) measuring an electrical signal at each of multiple locations in the distribution of materials in response to the current flow; (iii) providing spatial information about the distribution of materials with respect to a first reference frame, the spatial information indicative of regions of different complex conductivity in the distribution of materials; and (iv) determining the position of the object with respect to the spatial information about the distribution of materials based on measured electrical signals and the spatial information. In certain embodiments, the object is a catheter inserted into a patient's heart cavity for cardiac mapping.

### SUMMARY OF THE INVENTION

There is provided, in accordance with some embodiments of the present invention, an apparatus including a first electrode, a second electrode, and circuitry. The circuitry is configured to obtain a measurement of an electrogram voltage between the first electrode and second electrode while the first electrode and second electrode are disposed within a body of a subject. The circuitry is further configured to, while obtaining the measurement, cause (i) a first current, which has a first frequency, to flow between the first electrode and one or more reference electrodes, and (ii) a second current, which has a second frequency, to flow between the second electrode and the reference electrodes, such that a beat voltage, which has a third frequency that is a difference between the first frequency and the second frequency and is within a frequency band of the electrogram voltage, is seen at the first electrode and the second electrode. The apparatus further includes an output-impedance-reducing coating, which coats the first electrode and second electrode so as to facilitate obtaining the measurement despite the beat voltage.

In some embodiments,
the circuitry is configured to cause the first current and second current to flow by generating the first current and second current, and
the output-impedance-reducing coating facilitates obtaining the measurement by reducing an amplitude of the beat voltage, relative to if the first electrode and second electrode were not coated.

In some embodiments,
the circuitry is configured to cause the first current to flow by applying a first applied voltage between the first electrode and the reference electrodes, and to cause the second current to flow by applying a second applied voltage between the second electrode and the reference electrodes, and
the output-impedance-reducing coating facilitates obtaining the measurement by reducing a minimum required amplitude of the first applied voltage and second applied voltage, relative to if the first electrode and second electrode were not coated.

In some embodiments, the circuitry is configured to cause the first current and second current to flow so as to facilitate ascertaining respective locations of the first electrode and second electrode.

In some embodiments, the circuitry is configured to cause the first current and second current to flow so as to facilitate ascertaining whether the first electrode contacts tissue of the subject and whether the second electrode contacts the tissue.

In some embodiments, the output-impedance-reducing coating includes a material selected from the group of materials consisting of: titanium nitride (TiN), iridium oxide, and a combination thereof.

In some embodiments, the output-impedance-reducing coating facilitates obtaining the measurement by facilitating an amplitude of the beat voltage that is less than 1 µV.

In some embodiments, the output-impedance-reducing coating includes a conductive polymer.

In some embodiments, the conductive polymer includes poly(3,4-ethylenedioxythiophene) polystyrene sulfonate (PEDOT:PSS) .

There is further provided, in accordance with some embodiments of the present invention, a method including obtaining a measurement of an electrogram voltage between a first electrode and a second electrode disposed within a body of a subject. The method further includes, while obtaining the measurement, causing (i) a first current, which has a first frequency, to flow between the first electrode and one or more reference electrodes, and (ii) a second current, which has a second frequency, to flow between the second electrode and the reference electrodes, such that a beat voltage, which has a third frequency that is a difference between the first frequency and the second frequency and is within a frequency band of the electrogram voltage, is seen at the first electrode and the second electrode. The first electrode and second electrode are coated with an output-impedance-reducing coating that facilitates obtaining the measurement despite the beat voltage.

In some embodiments, obtaining the measurement includes obtaining the measurement from a heart of the subject.

In some embodiments, obtaining the measurement includes obtaining the measurement from a brain of the subject.

The present invention will be more fully understood from the following detailed description of embodiments thereof, taken together with the drawings, in which:

### BRIEF DESCRIPTION OF THE DRAWINGS

Fig. 1 is a schematic illustration of an electrophysiological mapping system, in accordance with some embodiments of the present invention; and
Fig. 2 is a schematic illustration of circuitry, in accordance with some embodiments of the present invention.

### DETAILED DESCRIPTION OF EMBODIMENTS

### OVERVIEW

In an electrophysiological mapping procedure, an intrabody probe comprising a plurality of sensing electrodes at its distal end acquires electrogram signals from intrabody (e.g., intracardiac) tissue of a subject. Each signal is associated with the location at which the signal was acquired. Based on the association between the signals and the locations, a processor constructs a map of the tissue in which portions of the map are annotated so as to indicate electrophysiological properties of the tissue.

While the electrogram signals are acquired, electric current may be run between each of the sensing electrodes and reference electrodes coupled to the subject's body. Given that the impedance between each sensing electrode and each reference electrode varies with the location of the sensing electrode, such current facilitates computing the location of the sensing electrode. Alternatively or additionally, to ascertain whether each sensing electrode is in contact with the tissue, current may be run between the sensing electrode and another reference electrode coupled to the probe proximally to the sensing electrodes. To facilitate differentiating between the sensing electrodes, each sensing electrode may be assigned a different respective frequency, such that the frequency of the current between one sensing electrode and the reference electrodes is different from the frequency of the current between another sensing electrode and the reference electrodes.

A challenge, when running electric currents for location tracking and/or contact-ascertainment as described above, is that beat frequencies, which fall within the frequency band of the electrogram signals, may be generated. For example, a pair of sensing electrodes assigned respective frequencies of 97 kHz and 97.1 kHz may generate a beat frequency of 100 Hz. If the output impedance seen at the sensing electrodes is sufficiently high, the amplitude of these beat frequencies may be sufficiently high so as to interfere with the acquisition of the electrogram signals.

Given that the electrogram signals are acquired bipolarly by neighboring pairs of electrodes, one way to overcome this challenge is to assign the frequencies such that the minimum beat frequency generated by a neighboring pair of electrodes is greater than the maximum electrogram frequency. However, this constraint may be difficult to satisfy in practice, particularly as the positions of the electrodes relative to each other may change during the procedure. Another hypothetical solution is to pause the acquisition of electrogram signals while the currents are run. However, this would prolong the procedure; moreover, movement of an electrode might render inaccurate a previously-ascertained location or contact status for the electrode. Yet another hypothetical solution is to add one or more filter circuits for filtering out any beat frequencies; however, implementing this solution would increase the cost of the circuitry, and the filter circuits might add other types of noise to the electrogram-signal measurements.

Hence, embodiments of the present invention provide a superior solution, whereby the sensing electrodes are coated with a biocompatible coating that reduces the output impedance of the sensing electrodes. By virtue of the reduction in the output impedance, the voltage drop across each of the electrodes - and hence, the amplitudes of any generated beat signals - are sufficiently low so as not to interfere with the electrogram-signal acquisition. Thus, advantageously, the frequencies may be assigned without regard to the physical arrangement of the electrodes, and electrophysiological signals may be acquired by any neighboring pairs of electrodes while position tracking and/or contact sensing is performed. Moreover, no filter circuits are required.

In some embodiments, the coating comprises a metal such as titanium nitride (TiN) or iridium oxide. In other embodiments, the coating comprises a conductive polymer such as poly(3,4-ethylenedioxythiophene) polystyrene sulfonate (PEDOT:PSS).

### SYSTEM DESCRIPTION

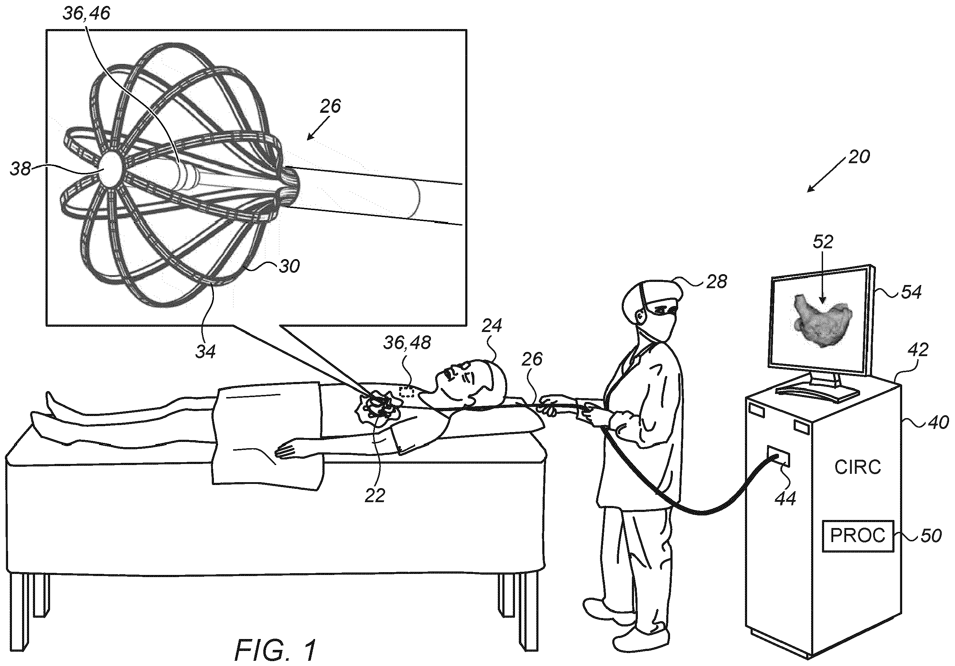

Reference is initially made to Fig. 1, which is a schematic illustration of an electrophysiological mapping system 20, in accordance with some embodiments of the present invention.

System 20 comprises an intrabody probe 26, comprising a plurality of sensing electrodes 34 at its distal end. Electrodes 34 may be made of gold, platinum, palladium, and/or any other suitable metal or metallic alloy.

Following the insertion of probe 26 into the body of a subject 24, a physician 28 navigates the distal end of the probe to the portion of the body that is to be mapped, such as a chamber of the heart 22 of subject 24. Subsequently, electrodes 34 are used to obtain measurements of electrogram voltages.

In the particular embodiment shown in Fig. 1, the distal end of the probe comprises a plurality of strips 30 to which the sensing electrodes are coupled. Strips 30 are distally coupled to a distal cap 38, such that the strips define a basket. In other embodiments, the distal end of the probe comprises a balloon to which the sensing electrodes are coupled. Alternatively, the distal end of the probe may have any other suitable form.

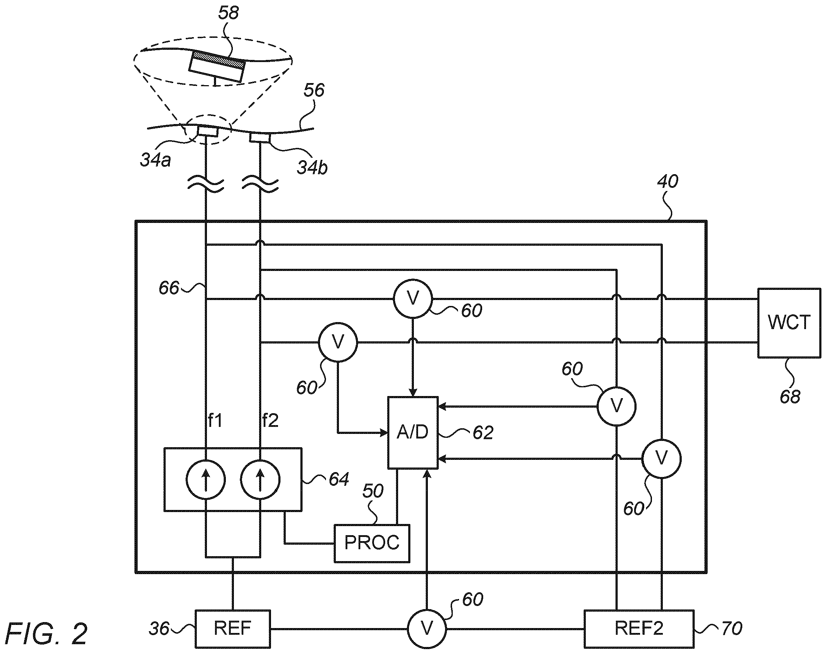

System 20 further comprises circuitry (CIRC) 40, which is typically contained within a console 42. Circuitry 40 is connected to the proximal end of probe 26, e.g., via an electrical interface 44 in console 42 such as a port or socket. Wires running through probe 26 transfer electrical signals between electrodes 34 and circuitry 40, as shown schematically in Fig. 2 (described below).

Typically, circuitry 40 comprises a processor 50 configured to perform at least some of the functionality described herein. Typically, the circuitry further comprises analog-to-digital (A/D) and digital-to-analog (D/A) conversion circuitry for interfacing between processor 50 and probe 26.

In general, the functionality of processor 50 may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, processor 50 may be embodied as a programmed processor comprising, for example, a central processing unit (CPU). Program code, including software programs, and/or data may be loaded for execution and processing by the CPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

As the distal end of the probe is moved within the subject, circuitry 40 causes electric currents to flow between each of the sensing electrodes and one or more reference electrodes 36, which are also wiredly connected to circuitry 40. These currents may facilitate ascertaining respective locations of the sensing electrodes, and/or whether each of the electrodes contacts tissue of the subject. Alternatively or additionally, the currents may facilitate performing any other suitable function.

For example, circuitry 40 may cause current to flow between each sensing electrode and one or more external reference electrodes 48 coupled to the subject's body. In particular, the circuitry may drive a current having a predefined amplitude between the electrode and reference electrodes 48, and then measure the resulting voltage between the electrode and each of the reference electrodes. Alternatively, the circuitry may apply a voltage having a predefined amplitude between the electrode and each of reference electrodes 48, and then measure the resulting current between the electrode and each of the reference electrodes. Based on the measured voltages or currents, the circuitry may compute the location of the electrode, as described, for example, in US Patents 5,983,126, 6,456,864, and 5,944,022, whose respective disclosures are incorporated herein by reference. Alternatively, the circuitry may calibrate a position map that maps the measured voltages or currents to the location of the electrode as indicated by an electromagnetic tracking system, as described, for example, in US Patent 7,536,218 to Govari et al. and US Patent 8,456,182 to Bar-Tal et al., whose respective disclosures are incorporated herein by reference. Alternatively, the circuitry may use the measured voltages or currents to look up the location in a calibrated position map.

Alternatively or additionally, the circuitry may cause current to flow between the electrode and an internal reference electrode 46 coupled to the probe. In particular, the circuitry may drive a current having a predefined amplitude between the electrode and reference electrode 46, and then measure the resulting voltage. Alternatively, the circuitry may apply a voltage having a predefined amplitude between the electrode and reference electrode 46, and then measure the resulting current. Subsequently, based on the measured voltage or current, the circuitry may compute the impedance between the electrode and the reference electrode. Based on the impedance (or based directly on the measured voltage or current), the circuitry may ascertain whether the electrode contacts tissue of the subject. (In general, a higher impedance indicates contact with tissue, while a lower impedance indicates lack of contact.)

In addition to causing current to flow between the sensing electrodes and reference electrodes, circuitry 40 obtains measurements of electrogram voltages, as further described below with reference to Fig. 2. Based on the electrogram voltages, the circuitry may construct an electrophysiological map 52 and, optionally, display map 52 on a display 54.

Although Fig. 1 relates mainly to heart electrograms, it is noted that embodiments of the present invention may also be applied to the acquisition of brain electrograms, e.g., during a neurosurgical procedure.

Reference is now made to Fig. 2, which is a schematic illustration of circuitry 40, in accordance with some embodiments of the present invention.

In the scenario depicted schematically in Fig. 2, circuitry 40 obtains a measurement of an electrogram voltage between a first sensing electrode 34a and a second sensing electrode 34b that contact tissue 56 within the body of subject 24 (Fig. 1). First electrode 34a and second electrode 34b typically neighbor one another on probe 26; for example, first electrode 34a and second electrode 34b may be disposed next to one another on a single strip 30 (Fig. 1).

Typically, the electrogram voltage between the two electrodes is not measured directly. Rather, a voltage-difference-measuring element 60 measures the difference in voltage, within the frequency band of the electrogram voltage (e.g., 0.05 - 500 Hz), between the first sensing electrode and a common electrode or Wilson Central Terminal (WCT) 68. Similarly, another voltage-difference-measuring element 60 measures the difference in voltage, within the frequency band of the electrogram voltage, between the second electrode and the common electrode or WCT 68. (The first and second voltage-difference-measuring elements, along with the other voltage-difference-measuring elements shown in Fig. 2, may be embodied as different respective channels in a single voltage-difference-measuring device.) The analog signals representing the voltages are digitized by an A/D converter 62, and the digitized signals are passed to processor 50. Processor 50 computes the difference between the two voltages, thus obtaining the measurement of the electrogram voltage.

While the measurement is obtained, circuitry 40 causes (i) a first current, which has a first frequency f1, to flow between the first electrode and reference (REF) electrodes 36, and (ii) a second current, which has a second frequency f2, to flow between the second electrode and the reference electrodes. As described above with reference to Fig. 1, the currents may facilitate ascertaining the locations of the electrodes, and/or ascertaining whether the electrodes contact tissue 56.

So as not to corrupt the measurement of the electrogram voltage with noise, f1 and f2 are typically much larger than the highest frequency in the frequency band of the electrogram voltage; for example, each of f1 and f2 may be greater than 90 kHz. However, the difference between f1 and f2 - and hence, any beat frequency generated from the currents - may be within the frequency band. Hence, each of the electrodes is coated with an output-impedance-reducing coating 58, which facilitates obtaining the electrogram-voltage measurement despite any beat voltage having the beat frequency |f1 - f2|. Coating 58 may comprise titanium nitride (TiN), iridium oxide, a combination of TiN and iridium oxide, poly(3,4-ethylenedioxythiophene) polystyrene sulfonate (PEDOT:PSS), another conductive polymer, and/or any other suitable material.

In some embodiments, the circuitry causes the currents to flow by generating the currents; for example, the currents may be generated by a current source 64 controlled by processor 50. The resulting voltage between each sensing electrode and another reference electrode 70 (identified in Fig. 2 as "REF2") is measured, digitized, and received by the processor. (Reference electrode 70, which may be located on the probe, may comprise a TRUEREF^{®} electrode of Biosense Webster, Inc.) Likewise, the respective voltage between each of reference electrodes 36 and reference electrode 70 is measured, digitized, and received by the processor. Based on the received voltages, the processor calculates the respective voltage between each sensing electrode and each reference electrode.

In such embodiments, coating 58 facilitates obtaining the electrogram-signal measurement by reducing the amplitude of the beat voltage, relative to if the first electrode and second electrode were not coated. In other words, by virtue of the lower output impedance of (and hence, lower voltage drop across) the sensing electrodes, the beat voltage has a lower amplitude.

For example, each of the currents may be generated with an amplitude of at least 10 nA. Such currents, in the absence of coating 58, may cause each of the voltages on the wires 66 that connect the sensing electrodes to the reference electrodes to have an amplitude of at least 1 µV. Thus, the amplitude of the beat voltage seen at the sensing electrodes may be at least 2 µV, which is large enough to corrupt the electrogram measurement of the sensing electrodes with noise. On the other hand, with coating 58, the amplitude of each of the voltages on wires 66 may be substantially lower, e.g., approximately 500 nV or, preferably, less than 500 nV, such that the amplitude of the beat voltage seen at the sensing electrodes may be approximately 1 µV or, preferably, less than 1 µV, which is sufficiently low so as not to corrupt the electrogram measurement.

In other embodiments, the circuitry causes the currents to flow by applying a first voltage between the first electrode and the reference electrodes, and a second voltage between the second electrode and the reference electrodes. Such voltages may be applied, for example, by a voltage source controlled by processor 50. The resulting currents flowing through the reference electrodes are measured, digitized, and received by the processor.

In such embodiments, coating 58 facilitates obtaining the measurement by reducing the minimum required amplitude of the applied voltages, relative to if the first electrode and second electrode were not coated. (In this context, the "minimum required" amplitude is the minimum amplitude required to perform whichever function is facilitated by the currents, such as ascertaining the respective locations of the electrodes.)

For example, it may be required that the amplitude of each of the currents on wires 66 be at least 10 nA. Such currents, in the absence of coating 58, may require that the amplitude of each of the applied voltages be at least 1 µV, such that the amplitude of the beat voltage is at least 2 µV. On the other hand, with coating 58, even a substantially lower applied-voltage amplitude, such as approximately 500 nV or, preferably, less than 500 nV, may be sufficient to cause a 10 nA current.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method, comprising:
   obtaining a measurement of an electrogram voltage between a first electrode and a second electrode disposed within a body of a subject; and
   while obtaining the measurement, causing (i) a first current, which has a first frequency, to flow between the first electrode and one or more reference electrodes, and (ii) a second current, which has a second frequency, to flow between the second electrode and the reference electrodes, such that a beat voltage, which has a third frequency that is a difference between the first frequency and the second frequency and is within a frequency band of the electrogram voltage, is seen at the first electrode and the second electrode,
      the first electrode and second electrode being coated with an output-impedance-reducing coating that facilitates obtaining the measurement despite the beat voltage.
2. The method according to aspect 1,
   wherein causing the first current and second current to flow comprises causing the first current and second current to flow by generating the first current and second current, and
   wherein the output-impedance-reducing coating facilitates obtaining the measurement by reducing an amplitude of the beat voltage, relative to if the first electrode and second electrode were not coated.
3. The method according to aspect 1,
   wherein causing the first current to flow comprises causing the first current to flow by applying a first applied voltage between the first electrode and the reference electrodes,
   wherein causing the second current to flow comprises causing the second current to flow by applying a second applied voltage between the second electrode and the reference electrodes, and
   wherein the output-impedance-reducing coating facilitates obtaining the measurement by reducing a minimum required amplitude of the first applied voltage and second applied voltage, relative to if the first electrode and second electrode were not coated.
4. The method according to aspect 1, wherein causing the first current and second current to flow comprises causing the first current and second current to flow so as to facilitate ascertaining respective locations of the first electrode and second electrode.
5. The method according to aspect 1, wherein causing the first current and second current to flow comprises causing the first current and second current to flow so as to facilitate ascertaining whether the first electrode contacts tissue of the subject and whether the second electrode contacts the tissue.
6. The method according to aspect 1, wherein the output-impedance-reducing coating includes a material selected from the group of materials consisting of: titanium nitride (TiN), iridium oxide, and a combination thereof.
7. The method according to aspect 1, wherein the output-impedance-reducing coating facilitates obtaining the measurement by facilitating an amplitude of the beat voltage that is less than 1 µV.
8. The method according to aspect 1, wherein the output-impedance-reducing coating includes a conductive polymer.
9. The method according to aspect 8, wherein the conductive polymer includes poly(3,4-ethylenedioxythiophene) polystyrene sulfonate (PEDOT:PSS).
10. The method according to aspect 1, wherein obtaining the measurement comprises obtaining the measurement from a heart of the subject.
11. The method according to aspect 1, wherein obtaining the measurement comprises obtaining the measurement from a brain of the subject.

## Claims

1. Apparatus, comprising:
a first electrode;
a second electrode;
circuitry, configured to:
obtain a measurement of an electrogram voltage between the first electrode and second electrode while the first electrode and second electrode are disposed within a body of a subject, and
while obtaining the measurement, cause (i) a first current, which has a first frequency, to flow between the first electrode and one or more reference electrodes, and (ii) a second current, which has a second frequency, to flow between the second electrode and the reference electrodes, such that a beat voltage, which has a third frequency that is a difference between the first frequency and the second frequency and is within a frequency band of the electrogram voltage, is seen at the first electrode and the second electrode; and
an output-impedance-reducing coating, which coats the first electrode and second electrode so as to facilitate obtaining the measurement despite the beat voltage.

2. The apparatus according to claim 1,
wherein the circuitry is configured to cause the first current and second current to flow by generating the first current and second current, and
wherein the output-impedance-reducing coating facilitates obtaining the measurement by reducing an amplitude of the beat voltage, relative to if the first electrode and second electrode were not coated.

3. The apparatus according to claim 1,
wherein the circuitry is configured to cause the first current to flow by applying a first applied voltage between the first electrode and the reference electrodes, and to cause the second current to flow by applying a second applied voltage between the second electrode and the reference electrodes, and
wherein the output-impedance-reducing coating facilitates obtaining the measurement by reducing a minimum required amplitude of the first applied voltage and second applied voltage, relative to if the first electrode and second electrode were not coated.

4. The apparatus according to claim 1, wherein the circuitry is configured to cause the first current and second current to flow so as to facilitate ascertaining respective locations of the first electrode and second electrode.

5. The apparatus according to claim 1, wherein the circuitry is configured to cause the first current and second current to flow so as to facilitate ascertaining whether the first electrode contacts tissue of the subject and whether the second electrode contacts the tissue.

6. The apparatus according to claim 1, wherein the output-impedance-reducing coating comprises a material selected from the group of materials consisting of: titanium nitride (TiN), iridium oxide, and a combination thereof.

7. The apparatus according to claim 1, wherein the output-impedance-reducing coating facilitates obtaining the measurement by facilitating an amplitude of the beat voltage that is less than 1 µV.

8. The apparatus according to claim 1, wherein the output-impedance-reducing coating comprises a conductive polymer.

9. The apparatus according to claim 8, wherein the conductive polymer comprises poly(3,4-ethylenedioxythiophene) polystyrene sulfonate (PEDOT:PSS).
